# EUROPÄISCHE PATENTSCHRIFT

(11) **EP 3 386 441 B1**
(45) Veröffentlichungstag und Bekanntmachung des Hinweises auf die Patenterteilung: **21.10.2020**
(21) Anmeldenummer: 16819292.0
(22) Anmeldetag: 12.12.2016
(51) Int. Cl.: A61F 2/30, A61F 2/36

(54) **KÜNSTLICHES GELENKIMPLANTAT UND HÜFTGELENKENDOPROTHESE**
ARTIFICIAL JOINT IMPLANT AND HIP-JOINT ENDOPROSTHESIS
IMPLANT D'ARTICULATION ARTIFICIELLE ET ENDOPROTHÈSE DE LA HANCHE

(30) Priorität: 11.12.2015 DE 102015121658
(43) Veröffentlichungstag der Anmeldung: 17.10.2018
(73) Patentinhaber: Aesculap AG, 78532 Tuttlingen (DE)
(72) Erfinder: HERMLE, Thomas, 78628 Rottweil (DE)
(74) Vertreter: Hoeger, Stellrecht & Partner Patentanwälte mbB
(86) Internationale Anmeldenummer: PCT/EP2016/080563
(87) Internationale Veröffentlichungsnummer: WO 2017/098034

(56) Entgegenhaltungen:
- DE-A1-102008 030 260
- DE-B3-102005 052 699
- DE-U1-202011 103 010

## Beschreibung

Die Erfindung betrifft ein künstliches Gelenkimplantat mit einem ersten Implantatteil, einem zweiten Implantatteil und mindestens einem Befestigungselement zum kraft- und/oder formschlüssigen Festlegen des ersten und des zweiten Implantatteils aneinander in einer Verbindungsstellung.

Ferner betrifft die vorliegende Erfindung eine Hüftgelenkendoprothese umfassend eine Gelenkpfanne und einen Hüftschaft, welcher einen mit der Gelenkpfanne zusammenwirkenden Gelenkkopf trägt oder mit einem solchen koppelbar ist.

Künstliche Gelenkimplantate der eingangs beschriebenen Art sind in vielfältiger Weise bekannt. Beispielsweise kann es sich dabei um Komponenten von Hüftgelenkendoprothesen oder Kniegelenkendoprothesen handeln.

Insbesondere bei modularen Prothesensystemen ist es üblich, erste und zweite Implantatteile mit mindestens einem Befestigungselement in einer Verbindungsstellung aneinander festzulegen. Beispielsweise werden Schultern an Hüftgelenksschäften vom Schaft selbst separat ausgebildet und nach dem Einführen des Schafts in eine Kavität des Femurs mit dem Schaft verbunden, beispielsweise durch Verschrauben. Hierfür wird üblicherweise mindestens ein Befestigungselement benutzt.

In der DE 20 2011 103 010 U1 sind Gelenkimplantate beschrieben. Implantate und Spannglieder für Implantate sind aus der DE 10 2005 052 699 B3 bekannt. Die DE 10 2008 030 260 A1 offenbart ein modulares Probeimplantatsystem.

Bei den bekannten modularen Systemen ist jedoch die Verbindung des ersten und zweiten Implantatteils mit dem mindestens einen Befestigungselement aufwendig.

Es ist daher eine Aufgabe der vorliegenden Erfindung, eine Implantation eines künstlichen Gelenkimplantats der eingangs beschriebenen Art zu vereinfachen.

Diese Aufgabe wird bei einem künstlichen Gelenkimplantat der eingangs beschriebenen Art erfindungsgemäß dadurch gelöst, dass das mindestens eine Befestigungselement und das erste Implantatteil unlösbar beweglich miteinander gekoppelt sind derart, dass das mindestens eine Befestigungselement nur durch Zerstören des mindestens einen Befestigungselements oder des ersten Implantatteils vom ersten Implantatteil lösbar ist, und dass das mindestens eine Befestigungselement einstückig ausgebildet ist.

Die erfindungsgemäß vorgeschlagene Lösung ermöglicht eine einfachere Implantation des Gelenkimplantats. Das mindestens eine Befestigungselement ist beweglich mit dem ersten Implantatteil gekoppelt, jedoch nicht von diesem lösbar. Damit kann es nicht verloren gehen. Insbesondere dann, wenn das mindestens eine Befestigungselement in Form einer Schraube oder einer Mutter ausgebildet ist, die im Vergleich zur Größe der miteinander zu verbindenden Implantatteile eher klein sind, ist es vorteilhaft, wenn sich das mindestens eine Befestigungselement nicht unbeabsichtigt vom ersten Implantatteil lösen und verloren gehen kann. Zudem können die Implantteile platzsparend ausgebildet werden. Auch eine Positionierung mehrerer Teile des Gelenkimplantats vereinfacht sich operativ. Außerdem kann als positive Folge eine Operationszeit reduziert werden. Durch die unlösbare Kopplung des mindestens einen Befestigungselements und des Implantatteils kann zudem die Zahl der von einem Operateur handzuhabenden Teile des Gelenkimplantats verringert werden. Gemäß der Erfindung ist das mindestens eine Befestigungselement einstückig ausgebildet ist. Auf diese Weise muss nur eine minimale Anzahl von Teilen beim Implantieren des Gelenkimplantats miteinander verbunden werden. Dies vereinfacht die Handhabung und hilft, eine Operationszeit zu reduzieren

Vorzugsweise sind das mindestens eine Befestigungselement und das erste Implantatteil relativ zueinander verdrehbar und/oder verschiebbar miteinander gekoppelt. Beispielsweise kann eine Mutter oder eine Schraube ein erstes Befestigungselement bilden, welches relativ zum ersten Implantatteil verschiebbar und/oder verdrehbar am ersten Implantatteil gehalten ist. Beispielsweise kann so die Schraube oder die Mutter relativ zum ersten Implantatteil verdreht und/oder verschoben werden, so dass sie mit einem entsprechenden zweiten Befestigungselement, beispielsweise einer Innengewindebohrung oder einem Gewindebolzen, verschraubt werden kann um das erste und das zweite Implantatteil in der Verbindungsstellung aneinander festzulegen.

Gemäß einer weiteren Ausführungsform der Erfindung kann das Gelenkimplantat eine Koppeleinrichtung umfassen zum beweglichen Koppeln des mindestens einen Befestigungselements und des ersten Implantatteils in einer Koppelstellung, welche Koppeleinrichtung zusammenwirkende erste und zweite, in der Koppelstellung miteinander in Eingriff stehende Koppelelemente aufweist, welche Koppelelemente einerseits am ersten Implantatteil und andererseits am mindestens einen Befestigungselement angeordnet oder ausgebildet sind. Mit einer derartigen Koppeleinrichtung lassen sich Befestigungselemente und Implantatteile auf einfache Weise miteinander in Eingriff bringen, so dass sie relativ zueinander beweglich und doch unlösbar aneinander gehalten sind. Unlösbar aneinander gehalten heißt hier wiederum, dass ein Lösen der Teile voneinander nur durch Zerstören eines oder beider Teile möglich ist.

Vorteilhaft ist es, wenn das erste Koppelement in Form einer Koppelausnehmung ausgebildet ist und dass das zweite Koppelelement in Form eines zur Koppelausnehmung korrespondierenden Koppelvorsprungs ausgebildet ist. Zum beweglichen Koppeln des ersten Implantatteils und des mindestens einen Befestigungselements stehen die ersten und zweiten Koppelelemente insbesondere in der beschriebenen Weise in Eingriff.

Auf besonders einfache und kompakte Weise ausbilden lässt sich das Gelenkimplantat, wenn am ersten Implantatteil eine Durchbrechung ausgebildet ist und wenn die Durchbrechung die Koppelausnehmung bildet. Beispielsweise kann es sich dabei um eine Bohrung handeln, in die das mindestens eine Befestigungselement oder ein Teil desselben in der Koppelstellung eingreift.

Günstig ist es, wenn am ersten und/oder am zweiten Koppelelement mindestens eine Hinterschneidung angeordnet oder ausgebildet ist und wenn am jeweils anderen Koppelelement ein in die Hinterschneidung eingreifender Hinterschneidungsvorsprung angeordnet oder ausgebildet ist. Durch die mit dem Hinterschneidungsvorsprung zusammenwirkende Hinterschneidung kann eine unlösbare Verbindung des ersten und des zweiten Koppelelements in der Koppelstellung auf einfache Weise realisiert werden, wobei die Koppelelemente trotzdem relativ zueinander beweglich aneinander gehalten sein können.

Die Herstellung des Gelenkimplantats wird besonders einfach, wenn die Hinterschneidung in Form einer Ringnut ausgebildet ist und wenn der Hinterschneidungsvorsprung in Form eines zur Ringnut korrespondierenden Ringvorsprungs ausgebildet ist. Der Ringvorsprung selbst muss nicht zwingend in sich geschlossen sein. Es können auch mehrere, ringförmig in Richtung auf eine Längsachse hinweisende oder von dieser weg weisende Vorsprünge vorgesehen sein, die eine sichere Kopplung der ersten und zweiten Koppelelemente in der Koppelstellung miteinander sicherstellen können.

Vorteilhaft ist es, wenn die Ringnut in Richtung auf eine Längsachse der Koppeleinrichtung hin weisend oder von dieser weg weisend geöffnet ausgebildet ist und wenn der Ringvorsprung von der Längsachse weg weisend oder auf diese hin weisend abstehend angeordnet oder ausgebildet ist. Derart ineinander greifende Koppelemente verhindern insbesondere ein Lösen des mindestens einen Befestigungselements und des ersten Implantatteils voneinander in einer Richtung parallel zur Längsachse.

Vorzugsweise definiert das erste und/oder das zweite Koppelelement die Längsachse. Beispielsweise können das erste und/oder das zweite Koppelelement rotationssymmetrisch ausgebildet sein und mit einer Symmetrieachse die Längsachse definieren.

Günstig ist es, wenn das erste und/oder das zweite Koppelelement rotationssymmetrisch oder im Wesentlichen rotationssymmetrisch zur Längsachse ausgebildet sind. So lassen sich die Koppelelemente und damit die Koppeleinrichtung insgesamt auf einfache Weise herstellen.

Um nur eine minimale Zahl an Teilen für das künstliche Gelenkimplantat bereitstellen zu müssen, ist es vorteilhaft, wenn das erste und das zweite Koppelelement jeweils einstückig ausgebildet sind. Zudem kann so eine besonders stabile Kopplung zwischen dem mindestens einen Befestigungselement und dem ersten Implantatteil erreicht werden, die sich nur lösen lässt durch Zerstören des mindestens einen Befestigungselement oder des ersten Implantatteils.

Vorteilhaft ist es, wenn das erste und/oder das zweite Koppelelement durch ein additives Herstellungsverfahren ausgebildet sind. Derart hergestellte erste und/oder zweite Koppelelemente können auf einfache Weise und in einem Arbeitsgang durch ein solches additives Herstellungsverfahren, das auch als generatives Herstellungsverfahren bezeichnet werden kann, ausgebildet werden. Zudem lassen sich eine Hinterschneidung und ein oder mehrere in diese eingreifende Hinterschneidungsvorsprünge ebenfalls in einem Arbeitsgang ausbilden. Eine aufwendige Montage, um das mindestens eine Befestigungselement und das erste Implantatteil miteinander unlösbar zu verbinden, ist dann nicht erforderlich.

Auf besonders einfache Weise herstellen lässt sich das Gelenkimplantat, wenn das erste und/oder das zweite Koppelelement durch ein 3D-Druckverfahren oder durch selektives Lasersintern ausgebildet sind. Insbesondere können auch das mindestens eine Befestigungselement und/oder das erste Implantatteil insgesamt durch die beschriebenen additiven Herstellungsverfahren ausgebildet sein.

Günstig ist es, wenn das erste Implantatteil und/oder das zweite Implantatteil einstückig ausgebildet ist. Auf diese Weise muss nur eine minimale Anzahl von Teilen beim Implantieren des Gelenkimplantats miteinander verbunden werden. Dies vereinfacht die Handhabung und hilft, eine Operationszeit zu reduzieren.

Gemäß einer weiteren bevorzugten Ausführungsform der Erfindung kann das Gelenkimplantat eine Verbindungseinrichtung umfassen zum kraft- und/oder formschlüssigen Verbinden des mindestens einen Befestigungselements und des zweiten Implantatteils miteinander in einer Verbindungsstellung. Durch diese Verbindungseinrichtung werden dann das erste und das zweite Implantatteil kraft- und/oder formschlüssig aneinander festgelegt.

Vorteilhaft ist es, wenn die Verbindungseinrichtung erste und zweite Verbindungselemente umfasst, die einerseits am mindestens einen Befestigungselement und andererseits am zweiten Implantatteil angeordnet oder ausgebildet sind und die in der Verbindungsstellung kraft- und/oder formschlüssig in Eingriff stehen und die in einer Trennstellung, in welcher das mindestens eine Befestigungselement und der zweite Implantatteil voneinander getrennt sind, außer Eingriff stehen. Eine derart ausgebildete Verbindungseinrichtung ermöglicht insbesondere auf einfache Weise eine kraft- und/oder formschlüssige Verbindung zwischen dem mindestens einen Befestigungselement und dem zweiten Implantatteil und damit auch zwischen dem ersten und dem zweiten Implantatteil.

Auf einfache Weise ausbilden lässt sich das Gelenkimplantat, wenn das erste Verbindungselement in Form eines Verbindungsvorsprungs und wenn das zweite Verbindungselement in Form einer Verbindungsaufnahme ausgebildet ist oder umgekehrt. Ein - beispielsweise am mindestens einen Befestigungselement ausgebildeter Verbindungsvorsprung - und eine korrespondierende Verbindungsaufnahme lassen sich auf einfache Weise miteinander in Eingriff bringen.

Auf einfache Weise herstellen lässt sich das Gelenkimplantat, wenn die Verbindungsaufnahme am zweiten Implantatteil in Form einer Durchbrechung oder in Form eines Sacklochs ausgebildet ist. Alternativ kann das zweite Verbindungselement auch in Form eines vorstehenden Zapfens oder Bolzens ausgebildet sein.

Auf einfache Weise lassen sich die Verbindungselemente miteinander in Eingriff bringen, wenn die ersten und zweiten Verbindungselemente miteinander verrast- und/oder verschraubbar ausgebildet sind. Eine Verrastung zwischen den Verbindungselementen ermöglicht insbesondere eine werkzeugfreie Verbindung zwischen dem mindestens einen Befestigungselement und dem zweiten Implantatteil. Eine verschraubbare Ausgestaltung hat insbesondere den Vorteil, dass das mindestens eine Befestigungselement und das zweite Implantatteil bei Bedarf auch wieder auf einfache Weise voneinander getrennt werden können.

Günstig ist es, wenn das erste Verbindungselement einen Außengewindeabschnitt und wenn das zweite Verbindungselement einen zum Außengewindeabschnitt korrespondierenden Innengewindeabschnitt aufweist. Derart ausgebildete Verbindungselemente ermöglichen auf einfache Weise das Verschrauben des mindestens einen Befestigungselements und des zweiten Implantatteils miteinander.

Um eine Handhabung des Gelenkimplantats bei der Implantation weiter zu verbessern, ist es günstig, wenn am Koppelelement des mindestens einen Befestigungselements eine Werkzeugelementaufnahme angeordnet oder ausgebildet ist zum in Eingriff Bringen mit einem Werkzeug, insbesondere einem Einschraubwerkzeug, zum Verbinden des mindestens einen Befestigungselements und des zweiten Implantatteils miteinander. Die Werkzeugelementaufnahme ermöglicht es insbesondere, mit einem entsprechenden Werkzeug das mindestens eine Befestigungselement mit dem zweiten Implantatteil kraft- und/oder formschlüssig in Eingriff zu bringen. Beispielsweise kann die Werkzeugelementaufnahme in Form eines Innenvielrunds ausgebildet sein.

Vorteilhaft ist es, wenn das erste Implantatteil und/oder das zweite Implantatteil und/oder das mindestens eine Befestigungselement aus einem körperverträglichen Metall und/oder Kunststoff hergestellt sind. Derartige Materialien zu wählen hat insbesondere den Vorteil, dass die Gefahr von Abstoßungsreaktionen des Körpers des Patienten minimiert ist.

Vorzugsweise ist das künstliche Gelenkimplantat in Form eines Prothesenschafts einer Hüftgelenkendoprothese ausgebildet. Alternativ kann das Gelenkimplantat auch in Form eines Tibiateils oder eines Femurteils einer Kniegelenkendoprothese oder in Form eines Prothesenschafts einer Schultergelenksprothese ausgebildet sein. Grundsätzlich kann das Gelenkimplantat zu einer beliebigen Endoprothese passen, wenn dieses modular aufgebaut ist und mindestens zwei miteinander zu verbindenden Implantatteile umfasst.

Günstig ist es, wenn der Prothesenschaft einen Schaftteil zum Einsetzen in eine Knochenkavität eines Femurs und ein Verschlusselement für eine Schaftteilöffnung des Schaftteils umfasst und wenn der Schaftteil den zweiten Implantatteil und das Verschlusselement den ersten Implantatteil definiert. Das Verschlusselement kann insbesondere in Form einer Schulter einer Revisionsprothese ausgebildet sein, die erst nach dem Einsetzen des Schaftteils in die Knochenkavität des Femurs mit dem Schaftteil verbunden wird. Das mindestens eine Befestigungselement kann insbesondere als ein rotierbarer Schraubbolzen oder eine rotierbar am Verschlusselement gehaltene Mutter ausgebildet sein, die mit dem Schaftteil kraft- und/oder formschlüssig verschraubt werden kann.

Günstigerweise ist der Schaftteil modular ausgebildet und weist einen Halsteil und einen Schaft auf, die in der Implantationsstellung kraft- und/oder formschlüssig in Eingriff stehen. Die modulare Ausbildung ermöglicht es, individuelle Längen des Schafts vorzusehen, um einen Patienten optimal versorgen zu können.

Die eingangs gestellte Aufgabe wird ferner bei einer Hüftgelenkendoprothese der eingangs beschriebenen Art erfindungsgemäß dadurch gelöst, dass der Hüftschaft in Form eines der oben beschriebenen künstlichen Gelenkimplantate ausgebildet ist.

Eine solche Hüftgelenkendoprothese weist dann insbesondere die oben im Zusammenhang mit bevorzugten Ausführungsformen von künstlichen Gelenkimplantaten beschriebenen Vorteile auf.

Die nachfolgende Beschreibung bevorzugter Ausführungsformen der Erfindung dienen im Zusammenhang mit den Zeichnungen der näheren Erläuterung. Es zeigen:
- Figur 1:: eine perspektivische Ansicht einer als Revisionsprothese ausgebildeten Hüftgelenkendoprothese mit einem künstlichen Gelenkimplantat in Form eines Hüftschafts;
- Figur 2:: eine teilweise durchbrochene Darstellung des künstlichen Gelenkimplantats aus Figur 1 mit entferntem Verschlusselement;

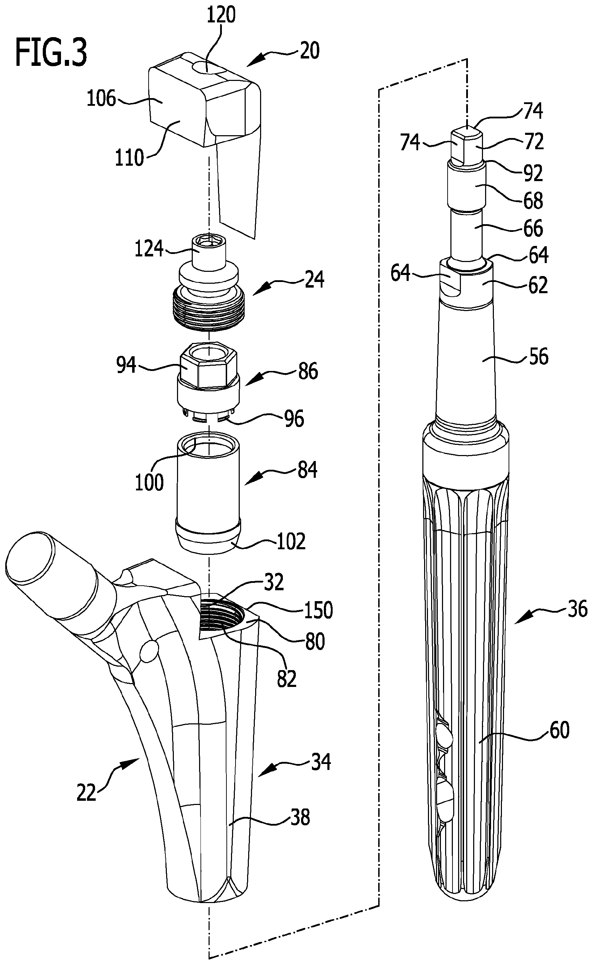

- Figur 3:: eine perspektivische Explosionsdarstellung des künstlichen Gelenkimplantats aus Figur 1;
- Figur 4:: eine teilweise Längsschnittansicht des künstlichen Gelenkimplantats aus Figur 1;
- Figur 5:: ein vergrößerter Ausschnitt der Längsschnittansicht aus Figur 4; und
- Figur 6:: eine Schnittansicht längs Linie 6-6 in Figur 5.

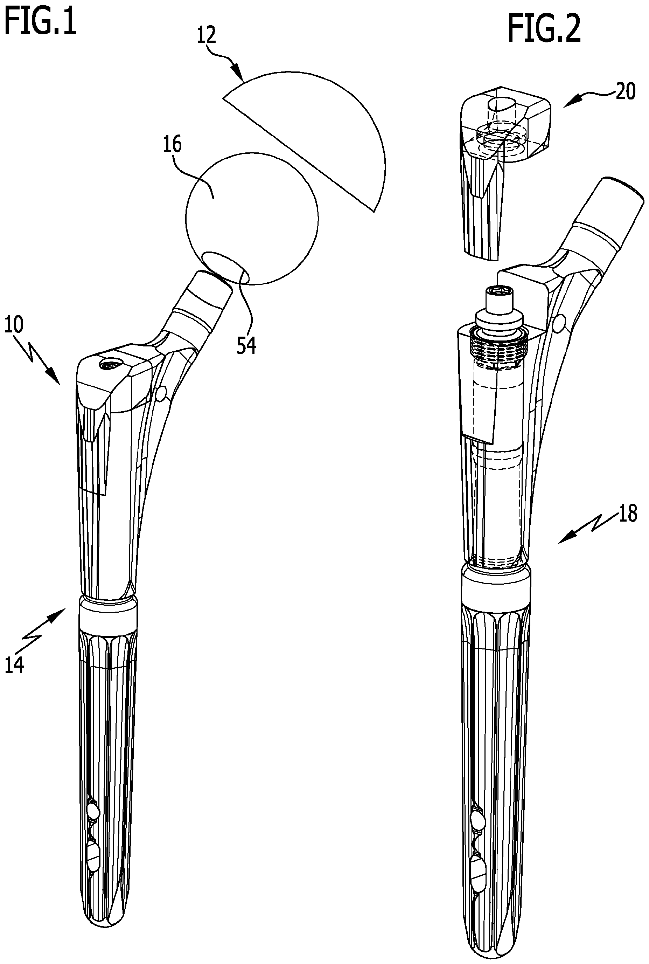

In Figur 1 ist beispielhaft eine Hüftgelenkendoprothese 10 schematisch dargestellt. Sie umfasst insbesondere eine Gelenkpfanne 12 und einen Hüftschaft 14, welcher mit einem mit der Gelenkpfanne 12 zusammenwirkenden Gelenckopf 16 koppelbar ist.

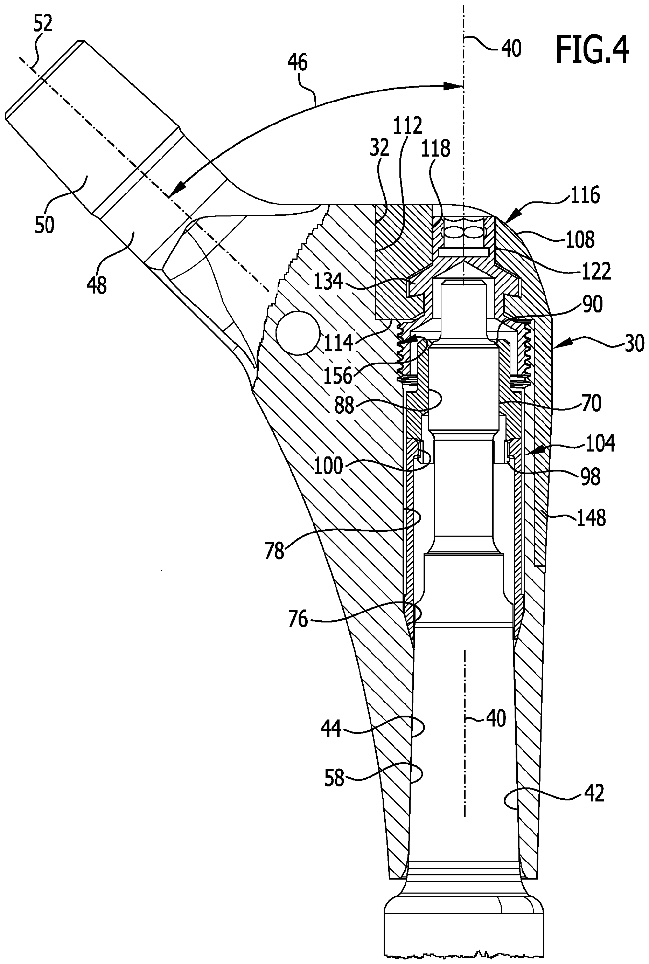

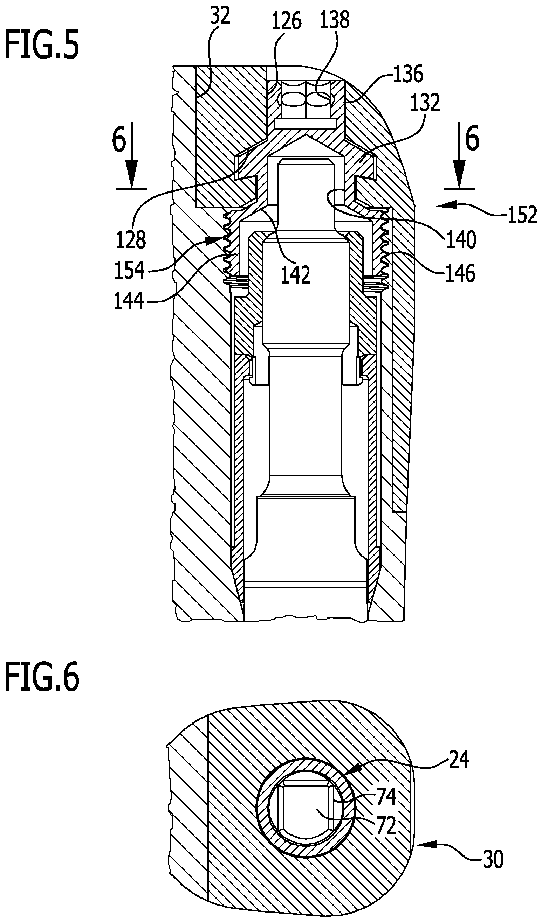

Der Hüftschaft 14 ist in Form eines künstlichen Gelenkimplantats 18 ausgebildet und umfasst einen ersten Implantatteil 20, einen zweiten Implantatteil 22 und ein Befestigungselement 24 zum kraft- und/oder formschlüssigen Festlegen des ersten und des zweiten Implantatteils aneinander in einer Verbindungsstellung, wie sie beispielsweise in den Figuren 1, 4, 5 und 6 schematisch dargestellt ist.

Der Hüftschaft 14 bildet einen Prothesenschaft 26 der Hüftgelenkendoprothese 10. Er umfasst einen Schaftteil 28 zum Einsetzen in eine Knochenkavität eines Femurs. Der Schaftteil 28 umfasst insbesondere den zweiten Implantatteil 22.

Ferner umfasst der Prothesenschaft ein Verschlusselement 30 für eine Schaftteilöffnung 32, die in Form einer Aussparung an einem Halsteil 34 des modular ausgebildeten Schaftteils 28 ausgebildet ist.

Der Schaftteil 28 umfasst ferner einen Schaft 36, welcher in einer Implantationsstellung mit dem Halsteil 34 kraft- und/oder formschlüssig in Eingriff steht oder verbunden ist. Die Implantationsstellung ist beispielsweise in den Figuren 1, 2, 4 und 5 schematisch dargestellt.

Der Halsteil 34 umfasst einen Schaftaufnahmeabschnitt 38, welcher einen eine Längsachse 40 definierenden, rotationssymmetrisch bezüglich der Längsachse 40 ausgebildeten Schaftaufnahme 42 in Form einer Durchbrechung 44 aufweist.

Vom Schaftaufnahmeabschnitt 38 steht unter einem Winkel 46 von etwa 45 Grad ein Halsabschnitt 48 ab, welcher ein freies, in Form eines Verbindungskonus 50 ausgebildetes freies Ende aufweist. Der Halsabschnitt 48 definiert eine Halslängsachse 52, die mit der Längsachse 40 den Winkel 46 einschließt. Korrespondierend zum Verbindungskonus 50 ist am Gelenkkopf 16 eine sich konisch in Richtung auf ein Zentrum des Gelenkkopfs 16 hin verjüngende Konusaufnahme ausgebildet. Der Gelenkkopf 16 und der Halsabschnitt 48 können so durch eine Presssitzverbindung verbunden werden.

Der Schaft 36 weist einen in die Schaftaufnahme 42 hineinragenden Konusabschnitt 56 auf, welcher zu einem Innenkonus 58 im Bereich der Schaftaufnahme 42 korrespondiert, so dass hier ebenfalls eine Presssitzverbindung ausgebildet werden kann. Ein in der Implantationsstellung distalseitig aus der Schaftaufnahme 42 vorstehender Schaftabschnitt 60 ist optional mit einer Profilierung versehen, um ein optimiertes Einwachsen in die Kavität des Femurs zu ermöglichen.

Zum Sichern des Schafts 36 in der Schaftaufnahme 42 ist proximalseitig des Konusabschnitts 56 am Schaft ein erster Werkzeugabschnitt 62 ausgebildet mit zwei parallel zueinander und zur Längsachse orientierten Abflachungen 64. Proximalseitig an den ersten Werkzeugabschnitt 62 schließt sich ein im Durchmesser verringerter zylindrischer Abschnitt 66 an. Etwas kürzer als der Abschnitt 66 ist ein sich an diesen proximalseitig anschließender zylindrischer Bolzenabschnitt 68, welcher mit einem Außengewinde 70 versehen ist. Ein proximales Ende des Schafts 36 bildet ein zweiter Werkzeugabschnitt 72, welcher ebenfalls zwei parallel zueinander und parallel zur Längsachse 40 verlaufende Abflachungen 74 aufweist.

Die Schaftaufnahme 42 erweitert sich unter Ausbildung einer konischen Schulter 76 im Innendurchmesser proximalseitig des Innenkonus 58 etwas und ist in Form einer zylindrischen Bohrung ausgebildet. Ausgehend von einem proximalen Ende 80 des Halsteils, welches die Schaftteilöffnung 32 begrenzt, ist in der Bohrung 78 ein kurzer Innengewindeabschnitt 82 ausgebildet.

Zum Verbinden des Schafts 36 mit dem Halsteil 34 dienen eine Klemmhülse 84 sowie eine Mutter 86. Die Mutter 86 weist ein Innengewinde 88 auf, welches zum Außengewinde 70 des Bolzenabschnitts 68 korrespondiert. Ferner ist ein proximales Ende der Mutter 86, welches sich an das Innengewinde 88 anschließt, etwas in Richtung auf die Längsachse 40 in der Art eines Flansches 90 vorspringend ausgebildet. Der Flansch 90 wirkt mit einer ringförmigen Anschlagfläche 92 im Übergangsbereich zwischen dem ersten Bolzenabschnitt 68 und dem zweiten Werkzeugabschnitt 72 zusammen.

Die Mutter 86 weist ein proximales, in Form eines Außensechskant 94 ausgebildetes Ende auf. Ein distales Ende der Mutter 86 ist in Form eines Hülsenabschnitts ausgebildet mit in distaler Richtung weisenden Rastelementen 96, welche von der Längsachse 40 weg weisende Rastnasen 98 tragen, die einen Richtung auf die Längsachse 40 hin weisenden Ringflansch 100 in der Klemmhülse 84 hintergreifen. Der Ringflansch 100 ist direkt am proximalen Ende der Klemmhülse 84 ausgebildet.

Distalseitig endet die Klemmhülse 84 in einer außen ausgebildeten konischen Klemmfläche 102, die zur Schulter 76 korrespondierend ausgebildet ist und sich an dieser abstützt, wenn der Schaft 36 mit dem Halsteil 34 verbunden ist.

Die Rastelemente 96 und der Ringflansch 100 bilden eine Rastverbindungseinrichtung 104 zum rastenden Verbinden der Klemmhülse 84 und der Mutter 86. Die Rastverbindungseinrichtung 104 ermöglicht es insbesondere, dass die Mutter 86 relativ zur Klemmhülse 84 um die Längsachse 40 verdreht werden kann. Die Klemmhülse 84 kann dann drehfest an der Schulter 76 anliegen und durch Verdrehen der Mutter 86 relativ zum Bolzenabschnitt 68 kann dann der Schaft 36 maximal weit in die Schaftaufnahme 42 hinein gezogen werden beziehungsweise die zwischen dem Konusabschnitt 56 und dem Innenkonus 58 ausgebildete Presssitzverbindung gesichert werden.

Der erste Implantatteil 20 bildet im Wesentlichen eine Schaftschulter 106, die eine abgerundete Außenfläche 108 sowie eine ebene Anlagefläche 110 an eine zu dieser korrespondierende, parallel zur Längsachse 40 und auf diese hin weisende Anlagefläche 112 der Schaftteilöffnung 32 aufweist.

Eine Unterseite der Schaftschulter 106 bildet ebenfalls eine Anlagefläche 114, die senkrecht zur Längsachse 40 verläuft und zur Anlage an das Ende 80 bestimmt ist.

Eine Besonderheit des Gelenkimplantats 18 ist insbesondere, dass das Befestigungselement 24 und das erste Implantatteil 20 unlösbar beweglich miteinander gekoppelt sind derart, dass das Befestigungselement 24 nur durch Zerstören desselben oder durch Zerstören des ersten Implantatteils 20 vom ersten Implantatteil lösbar ist.

Bei dem in den Figuren beispielhaft dargestellten Ausführungsbeispiel des Gelenkimplantats 18 sind das Befestigungselement 24 und das erste Implantatteil 20 relativ zueinander um die Längsachse 40 verdrehbar und minimal relativ zueinander verschiebbar in Richtung der Längsachse 40 miteinander gekoppelt.

Zum Koppeln des Befestigungselements 24 und des ersten Implantatteils 20 dient eine Koppeleinrichtung 116, die ein erstes Koppelelement 118 in Form einer Koppelausnehmung 120 umfasst. Ein zweites Koppelelement 122 ist in Form eines zur Koppelausnehmung 120 korrespondierend ausgebildeten Koppelvorsprungs 124 am Befestigungselement 24 ausgebildet.

Die Koppelausnehmung 120 wird definiert durch eine Durchbrechung 126 des ersten Implantatteils 20 koaxial zur Längsachse 40.

Um das erste Implantatteil 20 und das Befestigungselement 24 unlösbar beweglich aneinander zu halten, ist am ersten Implantatteil 20 eine Hinterschneidung 128 in Form einer in Richtung auf die Längsachse 40 hin geöffneten Ringnut 130 im Bereich der Durchbrechung 126 ausgebildet.

Am Befestigungselement 24 ist ein zur Hinterschneidung 128 korrespondierender und in diese eingreifender Hinterschneidungsvorsprung 132 ausgebildet in Form eines von der Längsachse 40 in radialer Richtung weg weisenden Ringvorsprungs 134.

Proximalseitig des Ringvorsprungs 134 ist am Befestigungselement 24 ein zylindrischer Endabschnitt 136 ausgebildet, welcher eine in proximaler Richtung weisend geöffnete Werkzeugelementaufnahme 138 aufweist, beispielsweise in Form eines Innenmehrkant oder eines Innenvielrund.

## Patentansprüche

1. Künstliches Gelenkimplantat (18) mit einem ersten Implantatteil (20), einem zweiten Implantatteil (22) und mindestens einem Befestigungselement (24) zum kraft- und/oder formschlüssigen Festlegen des ersten und des zweiten Implantatteils (20, 22) aneinander in einer Verbindungsstellung, **dadurch gekennzeichnet, dass** das mindestens eine Befestigungselement (24) und das erste Implantatteil (20) unlösbar beweglich miteinander gekoppelt sind derart, dass das mindestens eine Befestigungselement (24) nur durch Zerstören des mindestens einen Befestigungselements (24) oder des ersten Implantatteils (20) vom ersten Implantatteil (20) lösbar ist, und dass das mindestens eine Befestigungselement (24) einstückig ausgebildet ist.

2. Künstliches Gelenkimplantat nach Anspruch 1, **dadurch gekennzeichnet, dass** das mindestens eine Befestigungselement (24) und das erste Implantatteil (20) relativ zueinander verdrehbar und/oder verschiebbar miteinander gekoppelt sind.

3. Künstliches Gelenkimplantat nach Anspruch 1 oder 2, **gekennzeichnet durch** eine Koppeleinrichtung (116) zum beweglichen Koppeln des mindestens einen Befestigungselements (24) und des ersten Implantatteils (20) in einer Koppelstellung, welche Koppeleinrichtung (116) zusammenwirkende erste und zweite, in der Koppelstellung miteinander in Eingriff stehende Koppelelemente (118, 122) aufweist, welche Koppelelemente (118, 122) einerseits am ersten Implantatteil (20) und andererseits am mindestens einen Befestigungselement (24) angeordnet oder ausgebildet sind.

4. Künstliches Gelenkimplantat nach Anspruch 3, **dadurch gekennzeichnet, dass** das erste Koppelelement (118) in Form einer Koppelausnehmung (120) ausgebildet ist und dass das zweite Koppelelement (122) in Form eines zur Koppelausnehmung (120) korrespondierenden Koppelvorsprungs (124) ausgebildet ist
wobei insbesondere am ersten Implantatteil (20) eine Durchbrechung (126) ausgebildet ist und dass die Durchbrechung (126) die Koppelausnehmung (120) bildet.

5. Künstliches Gelenkimplantat nach Anspruch 3 oder 4, **dadurch gekennzeichnet, dass** am ersten und/oder am zweiten Koppelelement (118, 122) mindestens eine Hinterschneidung (128) angeordnet oder ausgebildet ist und dass am jeweils anderen Koppelelement (118, 122) ein in die Hinterschneidung (128) eingreifender Hinterschneidungsvorsprung (132) angeordnet oder ausgebildet ist.

6. Künstliches Gelenkimplantat nach Anspruch 5, **dadurch gekennzeichnet, dass** die Hinterschneidung (128) in Form einer Ringnut (130) ausgebildet ist und dass der Hinterschneidungsvorsprung (132) in Form eines zur Ringnut (130) korrespondierenden Ringvorsprungs (134) ausgebildet ist.

7. Künstliches Gelenkimplantat nach Anspruch 6, **dadurch gekennzeichnet, dass** die Ringnut (130) in Richtung auf eine Längsachse (40) der Koppeleinrichtung (116) hin weisend oder von dieser weg weisend geöffnet ausgebildet ist und dass der Ringvorsprung (134) von der Längsachse (40) weg weisend oder auf diese hin weisend abstehend angeordnet oder ausgebildet ist,
wobei insbesondere das erste und/oder das zweite Koppelelement (118, 122) die Längsachse (40) definiert,
wobei weiter insbesondere das erste und/oder das zweite Koppelelement (118, 122) rotationssymmetrisch oder im Wesentlichen rotationssymmetrisch zur Längsachse ausgebildet sind.

8. Künstliches Gelenkimplantat nach einem der Ansprüche 3 bis 7, **dadurch gekennzeichnet, dass** das erste und das zweite Koppelelement (118, 122)
a) jeweils einstückig ausgebildet sind
und/oder
b) durch ein additives Herstellungsverfahren ausgebildet sind und/oder
c) durch ein 3D-Druckverfahren oder durch selektives Lasersintern ausgebildet sind.

9. Künstliches Gelenkimplantat nach einem der voranstehenden Ansprüche, **dadurch gekennzeichnet, dass** das erste Implantatteil (20) und/oder das zweite Implantatteil (22) einstückig ausgebildet ist.

10. Künstliches Gelenkimplantat nach einem der voranstehenden Ansprüche, **gekennzeichnet durch** eine Verbindungseinrichtung (152) zum kraft- und/oder formschlüssigen Verbinden des mindestens einen Befestigungselements (24) und des zweiten Implantatteils (22) miteinander in einer Verbindungsstellung.

11. Künstliches Gelenkimplantat nach Anspruch 10, **dadurch gekennzeichnet, dass** die Verbindungseinrichtung (152) erste und zweite Verbindungselemente (154, 156) umfasst, die einerseits am mindestens einen Befestigungselement (24) und andererseits am zweiten Implantatteil (22) angeordnet oder ausgebildet sind und die in der Verbindungsstellung kraft- und/oder formschlüssig in Eingriff stehen und die in einer Trennstellung, in welcher das mindestens eine Befestigungselement (24) und der zweite Implantatteil (22) voneinander getrennt sind, außer Eingriff stehen.

12. Künstliches Gelenkimplantat nach Anspruch 11, **dadurch gekennzeichnet, dass**
a) das erste Verbindungselement (154) in Form eines Verbindungsvorsprungs (144) und dass das zweite Verbindungselement (156) in Form einer Verbindungsaufnahme (42) ausgebildet ist oder umgekehrt,
wobei insbesondere die Verbindungsaufnahme (42) am zweiten Implantatteil (22) in Form einer Durchbrechung (44) oder in Form eines Sacklochs ausgebildet ist
und/oder
b) die ersten und zweiten Verbindungselemente (154, 156) miteinander verrast- und/oder verschraubbar ausgebildet sind
und/oder
c) das erste Verbindungselement (154) einen Außengewindeabschnitt (146) und dass das zweite Verbindungselement (156) einen zum Außengewindeabschnitt (146) korrespondierenden Innengewindeabschnitt (82) aufweist.

13. Künstliches Gelenkimplantat nach einem der Ansprüche 3 bis 12, **dadurch gekennzeichnet, dass** am Koppelelement (122) des mindestens einen Befestigungselements (24) eine Werkzeugelementaufnahme (138) angeordnet oder ausgebildet ist zum in Eingriff Bringen mit einem Werkzeug, insbesondere einem Einschraubwerkzeug, zum Verbinden des mindestens einen Befestigungselements (24) und des zweiten Implantatteils (22) miteinander.

14. Künstliches Gelenkimplantat nach einem der voranstehenden Ansprüche, **dadurch gekennzeichnet, dass**
a) das erste Implantatteil (20) und/oder das zweite Implantatteil (22) und/oder das mindestens eine Befestigungselement (24) aus einem körperverträglichen Metall und/oder Kunststoff hergestellt sind und/oder
b) dass das Gelenkimplantat (18) in Form eines Prothesenschafts (26) einer Hüftgelenkendoprothese (10) ausgebildet ist,
wobei insbesondere der Prothesenschaft einen Schaftteil (28) zum Einsetzen in eine Knochenkavität eines Femurs und ein Verschlusselement (30) für eine Schaftteilöffnung (32) des Schaftteils (28) umfasst und dass der Schaftteil (28) den zweiten Implantatteil (22) und das Verschlusselement (30) den ersten Implantatteil (20) definiert,
wobei weiter insbesondere der Schaftteil (28) modular ausgebildet ist und einen Halsteil (34) und einen Schaft (36) aufweist, die in einer Implantationsstellung kraft- und/oder formschlüssig in Eingriff stehen.

15. Hüftgelenkendoprothese (10) umfassend eine Gelenkpfanne (12) und einen Hüftschaft (14), welcher einen mit der Gelenkpfanne zusammenwirkenden Gelenkkopf (16) trägt oder mit einem solchen koppelbar ist, **dadurch gekennzeichnet, dass** der Hüftschaft (14) in Form eines künstlichen Gelenkimplantats (18) nach einem der voranstehenden Ansprüche ausgebildet ist.

## Claims

1. Artificial joint implant (18), having a first implant part (20), a second implant part (22), and at least one fixing element (24) for fixing the first and the second implant part (20, 22) to each other in a non-positive- and/or positive-locking manner in a connecting position, **characterized in that** the at least one fixing element (24) and the first implant part (20) are moveably coupled to each other in a non-detachable manner, in such a way that the at least one fixing element (24) is detachable from the first implant part (20) only by destroying the at least one fixing element (24) or the first implant part (20), and **in that** the at least one fixing element (24) is integrally formed.

2. Artificial joint implant in accordance with Claim 1, **characterized in that** the at least one fixing element (24) and the first implant part (20) are coupled to each other so as to be twistable and/or displaceable relative to each other.

3. Artificial joint implant in accordance with Claim 1 or 2, **characterized by** a coupling device (116) for moveably coupling the at least one fixing element (24) and the first implant part (20) in a coupling position, which coupling device (116) has cooperative first and second coupling elements (118, 122) which are engaged with each other in the coupling position, which coupling elements (118, 122) are arranged or formed, on the one hand, on the first implant part (20) and, on the other hand, on the at least one fixing element (24).

4. Artificial joint implant in accordance with Claim 3, **characterized in that** the first coupling element (118) is configured in the form of a coupling recess (120), and **in that** the second coupling element (122) is configured in the form of a coupling projection (124) corresponding to the coupling recess (120),
wherein, in particular, a perforation (126) is formed on the first implant part (20), and **in that** the perforation (126) forms the coupling recess (120).

5. Artificial joint implant in accordance with Claim 3 to 4, **characterized in that** at least one undercut (128) is arranged or formed on the first and/or on the second coupling element (118, 122), and **in that** an undercut projection (132) engaging in the undercut (128) is arranged or formed on the respective other coupling element (118, 122).

6. Artificial joint implant in accordance with Claim 5, **characterized in that** the undercut (128) is configured in the form of an annular groove (130), and **in that** the undercut projection (132) is configured in the form of an annular projection (134) corresponding to the annular groove (130).

7. Artificial joint implant in accordance with Claim 6, **characterized in that** the annular groove (130) is formed open pointing in the direction toward a longitudinal axis (40) of the coupling device (116) or pointing away therefrom, and **in that** the annular projection (134) is arranged or formed protruding pointing away from the longitudinal axis (40) or pointing toward it,
wherein, in particular, the first and/or the second coupling element (118, 122) defines the longitudinal axis (40),
wherein further, in particular, the first and/or the second coupling element (118, 122) is formed rotationally symmetrically or substantially rotationally symmetrically to the longitudinal axis.

8. Artificial joint implant in accordance with any one of Claims 3 to 7, **characterized in that** the first and the second coupling element (118, 122)
a) are each integrally formed
and/or
b) are formed by way of an additive manufacturing method and/or
c) are formed by way of a 3D-printing method or by way of selective laser sintering.

9. Artificial joint implant in accordance with any one of the preceding Claims, **characterized in that** the first implant part (20) and/or the second implant part (22) is integrally formed.

10. Artificial joint implant in accordance with any one of the preceding Claims, **characterized by** a connecting device (152) for connecting the at least one fixing element (24) and the second implant part (22) to each other in a non-positive- and/or positive-locking manner in a connecting position.

11. Artificial joint implant in accordance with Claim 10, **characterized in that** the connecting device (152) comprises first and second connecting elements (154, 156) which are arranged or formed, on the one hand, on the at least one fixing element (24) and, on the other hand, on the second implant part (22), and which are engaged in a non-positive- and/or positive-locking manner in the connecting position, and which are disengaged in a separating position in which the at least one fixing element (24) and the second implant part (22) are separated from each other.

12. Artificial joint implant in accordance with Claim 11, **characterized in that**
a) the first connecting element (154) is configured in the form of a connecting projection (144), and the second connecting element (156) in the form of a connecting receiver (42) or vice versa,
wherein, in particular, the connecting receiver (42) is configured on the second implant part (22) in the form of a perforation (44) or in the form of a blind hole,
and/or
b) the first and second connecting elements (154, 156) are configured to be latchable and/or screwable to each other
and/or
c) the first connecting element (154) has an externally threaded section (146), and **in that** the second connecting element (156) has an internally threaded section (82) corresponding to the externally threaded section (146).

13. Artificial joint implant in accordance with any one of Claims 3 to 12, **characterized in that** a tool element receiver (138) is arranged or formed on the coupling element (122) of the at least one fixing element (24) for being brought into engagement with a tool, in particular a screwing-in tool, for connecting the at least one fixing element (24) and the second implant part (22) to each other.

14. Artificial joint implant in accordance with any one of the preceding Claims, **characterized in that**
a) the first implant part (20) and/or the second implant part (22) and/or the at least one fixing element (24) are produced from a biocompatible metal and/or plastics material
and/or
b) the joint implant (18) is configured in the form of a prosthesis
shank (26) of a hip joint endoprosthesis (10)
wherein, in particular, the prosthesis shank comprises a shank part (28) for insertion into a bone cavity of a femur and a closure element (30) for a shank part opening (32) of the shank part (28), and **in that** the shank part (28) defines the second implant part (22) and the closure element (30) the first implant part (20),
wherein further, in particular, the shank part (28) is modularly constructed and has a neck part (34) and a shank (36), which are engaged in a non-positive- and/or positive-locking manner in the implantation position.

15. Hip joint endoprosthesis (10) comprising a joint socket (12) and a hip shank (14), which bears a joint head (16) interacting with a joint socket or that is coupleable to such a joint head (16), **characterized in that** the hip shank (14) is configured in the form of an artificial joint implant (18) in accordance with any one of the preceding Claims.

## Revendications

1. Implant articulaire artificiel (18) avec une première partie d'implant (20), une deuxième partie d'implant (22) et au moins un élément de fixation (24) pour fixer par assemblage de force et/ou de forme les première et deuxième parties d'implant (20, 22) l'une à l'autre dans une position de liaison, **caractérisé en ce que** le au moins un élément de fixation (24) et la première partie d'implant (20) sont couplés l'un à l'autre de manière mobile et non séparable de sorte que le au moins un élément de fixation (24) ne peut être séparé de la première partie d'implant (20) que par destruction du au moins un élément de fixation (24) ou de la première partie d'implant (20), et **en ce que** le au moins un élément de fixation (24) est formé d'une seule pièce.

2. Implant articulaire artificiel selon la revendication 1, **caractérisé en ce que** le au moins un élément de fixation (24) et la première partie d'implant (20) sont couplés l'un à l'autre de manière à pouvoir tourner et/ou à être déplaçables l'un par rapport à l'autre.

3. Implant articulaire artificiel selon la revendication 1 ou 2, **caractérisé par** un dispositif de couplage (116) pour coupler de manière mobile le au moins un élément de fixation (24) et la première partie d'implant (20) dans une position de couplage, lequel dispositif de couplage (116) présente des premier et deuxième éléments de couplage (118, 122) qui sont en engagement l'un avec l'autre dans la position de couplage, lesquels éléments de couplage (118, 122) sont disposés ou formés d'une part sur la première partie d'implant (20) et d'autre part sur le au moins un élément de fixation (24).

4. Implant articulaire artificiel selon la revendication 3, **caractérisé en ce que** le premier élément de couplage (118) est formé sous forme d'un évidement de couplage (120) et **en ce que** le deuxième élément de couplage (122) est formé sous forme d'une saillie de couplage (124) correspondant à l'évidement de couplage (120), où en particulier une ouverture (126) est formée sur la première partie d'implant (20) et **en ce que** l'ouverture (126) forme l'évidement de couplage (120).

5. Implant articulaire artificiel selon la revendication 3 ou 4, **caractérisé en ce qu'**au moins une contre-dépouille (128) est disposée ou formée sur le premier et/ou sur le deuxième élément de couplage (118, 122) et **en ce qu'**une saillie de contre-dépouille (132) s'engageant dans la contre-dépouille (128) est disposée ou formée sur l'autre élément de couplage (118, 122).

6. Implant articulaire artificiel selon la revendication 5, **caractérisé en ce que** la contre-dépouille (128) est formée sous forme d'une rainure annulaire (130) et **en ce que** la saillie de contre-dépouille (132) est formée sous forme d'une saillie annulaire (134) correspondant à la rainure annulaire (130).

7. Implant articulaire artificiel selon la revendication 6, **caractérisé en ce que** la rainure annulaire (130) est formée ouverte en étant tournée dans la direction d'un axe longitudinal (40) du dispositif de couplage (116) ou à l'opposé de celui-ci, et **en ce que** la saillie annulaire (134) est disposée ou formée en saillie en étant tournée à l'opposé de l'axe longitudinal (40) ou vers lui,
où en particulier le premier et/ou le deuxième élément de couplage (118, 122) définit l'axe longitudinal (40),
où en particulier encore le premier et/ou le deuxième élément de couplage (118, 122) sont formés de manière symétrique en rotation ou sensiblement symétrique en rotation par rapport à l'axe longitudinal.

8. Implant articulaire artificiel selon l'une des revendications 3 à 7, **caractérisé en ce que** le premier et le deuxième élément de couplage (118, 122)
a) sont formés chacun d'une seule pièce
et/ou
b) sont formés par un procédé de fabrication additif et/ou
c) sont formés par un procédé d'impression 3D ou par frittage au laser sélectif.

9. Implant articulaire artificiel selon l'une des revendications précédentes, **caractérisé en ce que** la première partie d'implant (20) et/ou la deuxième partie d'implant (22) est formée d'une seule pièce.

10. Implant articulaire artificiel selon l'une des revendications précédentes, **caractérisé par** un dispositif de liaison (152) pour la liaison par assemblage de force et/ou de forme du au moins un élément de fixation (24) et de la deuxième partie d'implant (22) entre eux dans une position de liaison.

11. Implant articulaire artificiel selon la revendication 10, **caractérisé en ce que** le dispositif de liaison (152) comprend des premier et deuxième éléments de liaison (154, 156), qui sont disposés ou formés d'une part sur le au moins un élément de fixation (24) et d'autre part sur la deuxième partie d'implant (22) et qui sont en engagement par assemblage de force et/ou de forme dans la position de liaison et qui sont hors d'engagement dans une position de séparation dans laquelle le au moins un élément de fixation (24) et la deuxième partie d'implant (22) sont séparés l'un de l'autre.

12. Implant articulaire artificiel selon la revendication 11, **caractérisé en ce que**
a) le premier élément de liaison (154) est formé sous forme d'une saillie de liaison (144) et **en ce que** le deuxième élément de liaison (156) est formé sous forme d'un logement de liaison (42) ou vice versa,
où en particulier le logement de liaison (42) est formé sur la deuxième partie d'implant (22) sous forme d'une ouverture (44) ou sous forme d'un trou borgne,
et/ou
b) les premier et deuxième éléments de liaison (154, 156) sont formés de manière à pouvoir être verrouillés et/ou vissés ensemble et/ou
c) le premier élément de liaison (154) présente une section filetée extérieurement (146) et **en ce que** le deuxième élément de liaison (156) présente une section filetée intérieurement (82) correspondant à la section filetée extérieurement (146).

13. Implant articulaire artificiel selon l'une des revendications 3 à 12, **caractérisé en ce qu'**un logement d'élément d'outil (138) est disposé ou formé sur l'élément de couplage (122) du au moins un élément de fixation (24) pour s'engager avec un outil, en particulier un outil à visser, pour la liaison du au moins un élément de fixation (24) et de la deuxième partie d'implant (22) l'un à l'autre.

14. Implant articulaire artificiel selon l'une des revendications précédentes, **caractérisé en ce que**
a) la première partie d'implant (20) et/ou la deuxième partie d'implant (22) et/ou le au moins un élément de fixation (24) sont fabriqués en un métal et/ou plastique biocompatible
et/ou
b) l'implant articulaire (18) est formé sous forme d'une tige de prothèse (26) d'une endoprothèse d'articulation de la hanche (10),
où en particulier la tige de prothèse comprend une partie formant tige (28) pour l'insertion dans une cavité osseuse d'un fémur et un élément de fermeture (30) pour une ouverture de partie formant tige (32) de la partie formant tige (28) et **en ce que** la partie formant tige (28) définit la deuxième partie d'implant (22) et l'élément de fermeture (30) la première partie d'implant (20),
où en particulier encore la partie formant tige (28) est formée de manière modulaire et présente une partie col (34) et une tige (36) qui, dans une position d'implantation, sont en engagement par assemblage de force et/ou de forme.

15. Endoprothèse d'articulation de la hanche (10) comprenant une cupule d'articulation (12) et une tige de hanche (14) qui porte une tête d'articulation (16) coopérant avec la cupule d'articulation ou peut être couplée à celle-ci, **caractérisée en ce que** la tige de hanche (14) est formée sous forme d'un implant articulaire artificiel (18) selon l'une des revendications précédentes.
